# EUROPÄISCHE PATENTSCHRIFT

(11) **EP 1 224 926 B1**
(45) Veröffentlichungstag und Bekanntmachung des Hinweises auf die Patenterteilung: **17.11.2004**
(21) Anmeldenummer: 02001045.0
(22) Anmeldetag: 18.01.2002
(51) Int. Cl.: A61K 6/083, A61K 6/06

(54) **Dentalmaterialien auf der Basis von Metalloxid-Clustern**
Dental material containing metal oxide clusters
Matériau dentaire contenant des agglomérats d'oxyde métallique

(30) Priorität: 19.01.2001 DE 10102297
(43) Veröffentlichungstag der Anmeldung: 24.07.2002
(73) Patentinhaber: Ivoclar Vivadent AG, 9494 Schaan (LI)
(72) Erfinder: Moszner, Norbert, 9492 Eschen (LI); Völkel, Thomas, 88131 Oberreitnau (DE); Schubert, Ulrich, 2752 Wöllersdorf (AT); Rheinberger, Volker, 9490 Vaduz (LI)
(74) Vertreter: UEXKÜLL & STOLBERG

(56) Entgegenhaltungen:
- WO-A-00/69392
- DE-A- 3 137 840
- ULRICH SCHUBERT, GUIDO KICKELBICK, NICOLA HÜSING: "Nanoscale Structures of Sol-Gel Materials" MOLECULAR CRYSTALS AND LIQUID CRYSTALS SCIENCE AND TECHNOLOGY, SECTION A: MOLECULAR CRYSTALS AND LIQUID CRYSTALS, Bd. 354, 2000, Seiten 107-122, XP008002763
- ULRICH SCHUBERT ET AL: "INORGANIC-ORGANIC HYBRID POLYMERS FROM SURFACE-MODIFIED OXOMETALLATE CLUSTERS" MATERIALS RESEARCH SOCIETY SYMPOSIUM - PROCEEDINGS, Bd. 628, 24. April 2000 (2000-04-24), Seiten CC2.3.1-CC2.3.11, XP008002756
- ULRICH SCHUBERT, GUIDO KICKELBICK: "Oxozirconium Methacrylate Clusters: Zr6(OH)4O4(OMc)12 and Zr4O2(OMc)12 (OMc = Methacrylate)" CHEM. BER./RECUEIL, Bd. 130, 1997, Seiten 473-477, XP002198659

## Beschreibung

Die Erfindung betrifft Dentalmaterialien auf der Basis von polymerisierbaren Metalloxid-Clustern.

Polymerisierbare Zusammensetzungen, die neben organischen Monomeren polymerisierbare Metallverbindungen enthalten, sind bekannt.

Die US 2,502,411 offenbart Zusammensetzungen, die neben ungesättigten polymerisierbaren organischen Verbindungen ein Zirkonylacrylat enthalten, das durch Umsetzung eines wasserlöslichen Zirkoniumsalzes mit einem Salz der (Meth)acrylsäure erhältlich ist. Die Zirkoniumverbindung soll die Benetzbarkeit von Keramiken, Metallen und Cellulose verbessern. Angaben zur Struktur der Zirkonylacrylate werden nicht gemacht.

Die DE 31 37 840 C2 offenbart kristallines Zirkonmethacrylat der allgemeinen Formel Zr₄(MAS)₁₀O₂X₂(H₂O)₂₋₄, in der MAS das Anion der Methacrylsäure und X ein Anion aus der Gruppe Hydroxid, Alkoxid, Halogenid und Carboxylat ist. Die Verbindungen sollen sich als Vernetzungsmittel bei der Herstellung von Vinylpolymerisaten durch radikalische Polymerisation von Vinylmonomeren eignen.

Schubert et al., Chem. Mater. 4 (1992) 291, beschreiben die Herstellung und Charakterisierung von methacrylatmodifizierten Titan- und Zirkoniumalkoxiden, die durch Umsetzung der entsprechenden Metallalkoholate mit Methacrylsäure zugänglich sind, und Kickelbick und Schubert, Chem. Ber./Recueil 130 (1997) 473, von kristallinen Oxozirkoniummethacrylat-Clustern der Formeln Zr₆(OH)₄O₄(OMc)₁₂ und Zr₄O₂(OMc)₁₂ in denen OMc das Anion der Methacrylsäure ist.

WO 00 69 392A offenbart Dentalmaterialien, die Metalloxidpartikel mit einem relativ großen Partikeldurchmesser (mindestens 10 nm, vorzugsweise mindestens 50 nm enthalten.

Die DE 41 33 494 C2 offenbart Dentalharzmassen auf der Basis von polymerisierbaren Polysiloxanen, die durch hydrolytische Kondensation eines oder mehrerer Silane hergestellt werden, von denen mindestens eines durch einen 1,4,6-Trioxaspiro-[4,4]-nonan-Rest oder eine ethylenisch ungesättigte Gruppe substituiert ist.

Aus der DE 44 16 857 C1 sind hydrolysierbare und polymerisierbare Silane bekannt, die einen geradkettigen oder verzweigten organischen Rest mit mindestens einer C=C-Doppelbindung und 4 bis 50 Kohlenstoffatomen enthalten.

Die EP 1 022 012 A2 und die US 6,096,903 offenbaren Dentalmaterialien auf der Basis von polymerisierbaren und hydrolysierbaren methacrylatmodifizierten bzw. oxetangruppenhaltigen Silanen.

Silane des oben beschriebenen Typs lassen sich allein oder zusammen mit anderen hydrolytisch kondensierbaren Verbindungen zu anorganischen Netzwerken kondensieren, die dann über die in den Silanen enthaltenen C=C-Doppelbindungen durch ionische oder radikalische Polymerisation unter Ausbildung von anorganischorganischen Netzwerken gehärtet werden können. Der Erfindung liegt die Aufgabe zugrunde, Dentalmaterialien mit verbesserten mechanischen Eigenschaften zur Verfügung zu stellen.

Diese Aufgabe wird durch Dentalmaterialien gelöst, die mindestens einen Cluster gemäß der allgemeinen Formel (I)

[(M¹)ₐ(M²)_{b}O_{c}(OH)_{d}(OR)ₑ(L-Sp-Z)_{f}] (I)

enthalten, in der
- M¹, M²: unabhängig voneinander jeweils für ein Metallatom der III. oder V. Hauptgruppe oder der I. bis VIII. Nebengruppe des Periodensystems der Elemente stehen;
- R: eine Alkylgruppe mit 1 bis 6 Kohlenstoffatomen ist;
- L: eine koordinierende Gruppe mit 2 bis 6 komplexierenden Zentren ist;
- Sp: eine Spacergruppe ist oder entfällt;
- Z: eine polymerisationsfähige Gruppe ist;
- a: eine Zahl von 2 bis 20 ist;
- b: eine Zahl von 0 bis 10 ist;
- c: eine Zahl von 1 bis 30 ist;
- d, e: unabhängig voneinander jeweils eine Zahl von 0 bis 30 sind;
- f: eine Zahl von 2 bis 30 ist.

Die jeweiligen Werte der Indices a bis f können in Abhängigkeit von der Art, Zahl und Wertigkeit der Metalle und Liganden variieren. Dabei nehmen die Indices c, d, e und f vorzugsweise solche Werte an, daß die positiven Ladungen der Metalle M¹ und M² vollständig ausgegliche werden und der Cluster neutral ist. Der Cluster kann jedoch auch positiv oder negativ geladen sein. In diesem Fall wird die Ladung des Clusters durch geeignete Gegenionen, wie beispielsweise H⁺, Alkali- oder Erdalkalimetallionen, NH₄⁺, NR^{θ}₄⁺mit R^{θ} = Alkyl, insbesondere C₁- bis C₄-Alkyl, bzw. OH⁻ R¹-COO⁻ mit R¹ = Alkyl, vorzugsweise C₁- bis C₁₀-Alkyl, besonders bevorzugt C₁- bis C₄-Alkyl, oder Halogenid, vorzugsweise F⁻ oder Cl⁻, kompensiert. Die Cluster (I) weisen beispielsweise eine Ladung von -4 bis +4, insbesondere +1 bis +4 auf.

Die Gruppe L kann chelatisierend oder verbrückend sein, d.h. die komplexierenden Zentren der Gruppe L können mit dem gleichen Metallatom oder vorzugsweise mit zwei oder mehreren unterschiedlichen Metallatomen verbunden sein.

Der Ligand (L-Sp-Z) kann neutral sein oder eine negative Ladung aufweisen. Neutrale Liganden oder Liganden mit einer ein- bis dreifachen negativen Ladung sind bevorzugt.

Die im Cluster vorhandenen Liganden (L-Sp-Z) können gleich oder verschieden sein. Bevorzugt sind Cluster, die 1 bis 4, vorzugsweise 1 oder 2 Arten von Liganden (L-Sp-Z) enthalten. Beispielsweise können in dem Cluster Zr₄O₂(Methacrylat)₁₂ zwei Methacrylat-Liganden durch Allylacetoacetat ersetzt werden. Der resultierende Cluster hat die Formel Zr₄O₂(Methacrylat)₁₀(Allylacetoacetat)₂, wobei sowohl Methacrylat als auch Allylacetoacetat Liganden des Typs (L-Sp-Z) sind, d.h. der Cluster enthält zwei Arten von Liganden des Typs (L-Sp-Z).

Weiter bevorzugt sind Cluster, die nur einen geringen Anteil an Alkoxygruppen enthalten (d > e). Vorzugsweise ist e ≤ (a+b), besonders bevorzugt ist e = 0.

Unabhängig voneinander wählbare bevorzugte Definitionen für die einzelnen Variablen sind:
- M¹, M² =: unabhängig voneinander Ti und/oder Zr;
- R =: eine Alkylgruppe mit 1 bis 4 Kohlenstoffatomen, insbesondere 1 bis 2 Kohlenstoffatomen;
- L =: α-Hydroxycarboxylat (-CH(OH)-COO⁻), α-Aminocarboxylat (-CH(NH₂)-COO⁻), β-Diketonat ([-C(-O⁻)=CH-C(=O)R^{κ}]; mit R^{κ} = Alkyl, vorzugsweise C₁- bis C₆-Alkyl, besonders bevorzugt C₁- bis C₃-Alkyl, insbesondere Methyl, Sulfonat (-SO₃⁻) oder Phosphonat (-PO₃²⁻), besonders bevorzugt Carboxylat (-COO⁻);
- Sp =: eine Alkylengruppe mit 1 bis 18 Kohlenstoffatomen, eine Oxyalkylengruppe 1 bis 18 Kohlenstoffatomen und 0 bis 6 Sauerstoffatomen oder eine Arylengruppe mit 6 bis 14 Kohlenstoffatomen, wobei der Spacer Sp eine oder mehrere, vorzugsweise 0 bis 2 der Gruppen O, S, CO-O, O-CO, CO-NH, NH-CO, O-CO-NH, NH-CO-O, und NH enthalten kann;
besonders bevorzugt ist Sp eine Alkylengruppe mit 1 bis 6, insbesondere 1 bis 3 Kohlenstoffatomen oder entfällt;
- Z =: eine ethylenisch ungesättigte Gruppe, eine Epoxid-, Oxetan-, Vinylether-, 1,3-Dioxolan-, Spiroorthoester-, besonders bevorzugt eine Methacryl- und/oder Acrylgruppe;
- a =: 2 bis 11;
- b =: 0 bis 4.

Die Werte der Indices c, d, e und f korrelieren auch hier mit der Zahl und Ladung der Metallatome. Vorzugsweise nehmen sie solche Werte an, daß die Ladung des Clusters ausgeglichen ist. Typische Werte für c sind im Fall der bevozugen Cluster 1 bis 11, insbesondere 2 bis 5, für d und e 0 bis 10 und insbesondere 0 bis 8, für f 4 bis 20 und insbesondere 6 bis 15.

Gemäß einer besonders bevorzugten Ausführungsform ist M¹ = M². Ganz besonders bevorzugt sind Cluster, in denen M¹ und M² jeweils Zirkonium sind.

Die polymerisierbaren Gruppen Z sind vorzugsweise direkt oder durch einen kurzen Spacer über Carboxylatgruppen an die Metallzentren gebunden.

Besonders bevorzugte Liganden des Typs (L-Sp-Z) sind Acrylat, Methacrylat, Oleat, Allylacetoacetat und Acetoacetoxyethylmethacrylat.

Besonders bevorzugte Cluster sind:
Zr₆(OH)₄O₄(OMc)₁₂; Zr₄O₂(OMc)₁₂; Zr₆O₂(OC₄H₉)₁₀(OMc)₁₀; Ti₆O₄(OC₂H₅)₈(OMc)₈; Ti₄O₂(O-i-C₃H₇)₆(OMc)₆; Ti₄O₂(O-i-C₃H₇)₆(OMc)₆; Ti₉O₈(OC₃H₇)₄(OMc)₁₆; Zr₄Ti₂O₄(OC₄H₉)₂(OMc)₁₄; Zr₂Ti₄O₄(OC₄H₉)₆(OMc)₁₀; Zr₄Ti₄O₆(OBu)₄(OMc)₁₆ und Zr₆Ti₂O₆(OMc)₂₀
wobei OMc jeweils für eine Methacrylatgruppe steht. Ebenso bevorzugt sind die Cluster, die anstelle der Methacrylatgruppen Acrylatgruppen enthalten.

Die Clustere gemäß Formel (I) lassen sich durch Umsetzen von Metallalkoxiden mit geeigneten polymerisationsfähigen Liganden herstellen, gegebenenfalls unter Zugabe von Wasser. Beispielsweise ergibt die Umsetzung von Zirkonium(IV)-propoxid (Zr(O-C₃H₇)₄) mit einem vierfachen molaren Überschuß an Methacrylsäure (HOMc) Cluster der Zusammensetzung Zr₄O₂(OMc)₁₂ (G. Kickelbick, U. Schubert, Chem. Ber./Recueil 130 (1997) 473):

4 Zr(OC₃H₇)₄ + 14 HOMc - Zr₄O₂(OMc)₁₂ + 2 C₃H₇OMc + 14 C₃H₇OH

Darüberhinaus können geeignete Cluster durch den Austausch von Liganden gegen polymerisationsfähige Liganden hergestellt werden. Beispielsweise ergibt die Umsetzung von Titanoxid-Clustern TiₐO_{c}(OOCR^{α})ₑ mit ungesättigten Carbonsäuren (HOOCR^{β}) Cluster der Zusammensetzung TiₐO_{c}(OOCR^{α})ₑ₋ᵤ(OOCR^{β})ᵤ. Konkret ergibt die Umsetzung des Titancarboxylat-Clusters Ti₆O₄(OC₂H₅)₈(Acetat)₈ mit Methacrylsäure (HOMc) den Cluster Ti₆O₄(OC₂H₅)₈(Acetat)₈₋ᵤ(OMc)ᵤ.

Alternativ können geeignete Cluster durch Derivatisierung anorganischer Cluster erhalten werden. Beispielsweise ergibt die Umsetzung von SiW₁₁O₃₉⁸⁻ mit Trichlor- oder Triethoxysilanen R^{γ}SiQ₃ (Q= Cl oder OC₂H₅) in denen R^{γ} eine polymerisierbare Gruppe enthält, polymerisierbare Cluster der Zusammensetzung SiW₁₁O₃₉(OSi₂R₂)⁴⁻.

Die Cluster gemäß Formel (I) stellen Stoffe hoher Reaktivität dar, die allein oder vorzugsweise in Kombination mit anderen polymerisierbaren Komponenten durch Polymerisation zu mechanisch stabilen Schichten, Form- und Füllkörpern verarbeitet werden können. Diese zeichnen sich durch einen sehr geringen Anteil an durch Lösungsmittel herauslösbare Monomere und eine hohe Stabilität auch unter feuchten Bedingungen aus. Die mechanischen Eigenschaften der gehärteten Materialien werden durch Wasserlagerung nicht beeinträchtigt.

Zur Härtung werden die polymerisierbaren Cluster oder Mischungen der Cluster mit weiteren polymerisierbaren Komponenten vorzugsweise mit einem Initiator für die ionische oder radikalische Polymerisation versetzt. In Abhängigkeit von der Art des verwendeten Initiators kann die Polymerisation thermisch, durch UVoder sichtbares Licht initiiert werden. Außerdem können die Mischungen weitere Additive, wie beispielsweise Farbmittel (Pigmente oder Farbstoffe), Stabilisatoren, Aromastoffe, mikrobiozide Wirkstoffe, Weichmacher und/oder UV-Absorber enthalten. Die Cluster gemäß Formel (I) und deren Mischungen eignen sich insbesondere zur Verwendung als Dentalmaterialien oder zur Herstellung von Dentalmaterialien. Unter Dentalmaterialien werden vorzugsweise Adhäsive, Beschichtungsmaterialien, Zemente und insbesondere Füllungsmaterialien verstanden.

Die Cluster gemäß Formel (I) weisen aufgrund ihres hohen Molekulargewichts nur eine geringe Flüchtigkeit auf und lassen sich daher weitgehend unbedenklich verarbeiten. Durch die Größe und Gestalt sowie die Anzahl der polymerisierbaren Gruppen der Cluster können die Vernetzungsdichte und damit die mechanischen Eigenschaften wie E-Modul und Festigkeit und das Quellverhalten in organischen Lösungsmitteln der ausgehärteten Materialien gezielt eingestellt werden. Größe und Gestalt der Cluster wie auch die Zahl der polymerisierbaren Gruppen pro Metallatom sind durch Variation der Syntheseparameter kontrollierbar. Die Clustergröße und -gestalt wird maßgeblich durch das Verhältnis von Metalloxid zu Ligand im Eduktgemisch bestimmt, daneben aber auch durch die Art der Reste R in ein eingesetzten Alkoxiden M¹(OR)ₙ bzw. M²(OR)ₙ. Weiterhin lassen sich die mechanischen Eigenschaften wie Festigkeit und Flexibilität über den Abstand zwischen den Metallzentren und polymerisierbaren Resten, d.h. über die Länge der Spacergruppen -Sp-, beeinflussen.

Bei den Clustern gemäß Formel (I) sind die polymerisationsfähigen Gruppen an eine kompakte partikuläre Clusterstruktur fixiert. Dies hat zur Folge, daß bei der Polymerisation harte Produkte mit einer hohen Vernetzungsdichte erhalten werden. Die Cluster stellen dreidimensionale Moleküle mit definierter räumlicher Gestalt und Größe dar und erlauben bei der Copolymerisation mit anderen Komponenten die Einstellung einer für den jeweiligen Zweck optimalen Vernetzungsdichte. Die Gestalt der Cluster gewährleistet eine vollständige Einbindung der Cluster in das Polymernetzwerk. Sie stellt eine einheitliche Umgebung für alle polymerisierbaren organischen Liganden sicher, so daß diese bezüglich ihrer Reaktion mit organischen Co-Monomeren praktisch gleichwertig sind, was dazu führt, daß sich um jeden Liganden eine einheitliche Polymerstruktur ausbildet.

Darüber hinaus lassen sich durch Art und Anzahl der Metallatome die Abrasivität oder die optischen Eigenschaften wie beispielsweise die Brechzahl variieren.

Als weitere polymerisierbare Komponenten sind solche Stoffe bevorzugt, in denen die Cluster löslich sind, d.h. insbesondere flüssige Materialien. Hier kommen in erster Linie radikalisch oder ionisch polymerisierbare mono- und polyfunktionelle Verbindungen in Betracht, insbesondere polymerisierbare organische Monomere und Silane und Polysiloxane mit polymerisierbaren Gruppen sowie Mischungen dieser Verbindungen.

Als polymerisierbare organische Monomere sind ethylenisch ungesättigte organische Monomere, insbesondere monofunktionelle oder polyfunktionelle Methacrylate bevorzugt, die allein oder in Mischungen eingesetzt werden können. Als bevorzugte Beispiele für diese Verbindungen kommen Mono(meth)acrylate, wie Methyl-, Ethyl-, Butyl-, Benzyl-, Furfuryl, Phenyl(meth)acrylat, Isobutyl(meth)acrylat, Cyclohexyl(meth)acrylat, und mehrfunktionelle (Meth)acrylate, wie Tetraethylenglycoldi(meth)acrylat, Triethylenglycoldi(meth)acrylat, Diethylenglycoldi(meth)acrylat, Ethylenglycoldi(meth)acrylat, Polyethylenglycoldi(meth)acrylat, Butandioldi(meth)acrylat, Hexandioldi(meth)acrylat, 1,10-Decandioldi(meth)acrylat, 1,12-Dodecandioldi(meth)acrylat, Bisphenol-A-di(meth)acrylat, Trimethylolpropantri(meth)acrylat, 2,2-Bis-4-(3-methacryloxy-2-hydroxypropoxy)-phenylpropan (Bis-GMA), Pentaerythrittetra(meth)acrylat, sowie die Reaktionsprodukte aus Isocyanaten, insbesondere Di- und/oder Triisocyanaten, und OH-gruppenhaltigen (Meth)acrylaten in Frage. Beispiele dafür sind die Umsetzungsprodukte von 1 Mol 2,2,4-Trimethylhexamethylendiisocyanat mit 2 Mol 2-Hydroxyethylenmethacrylat (UDMA) oder 2 Mol Hydroxypropylmethacrylat sowie die Umsetzungsprodukte von 2 Mol Glycerindimethacrylat mit 1 Mol 2,2,4-Trimethylhexamethylendiisocyanat, Isophorondiisocyanat oder α,α,α',α'-Tetramethylxylylen-m-disocyanat. Dabei ist der Einsatz von polyfunktionellen (Meth)acrylaten besonders bevorzugt. Unter polyfunktionellen Verbindungen werden solche mit mehreren polymerisierbaren Gruppen verstanden.

Weiter bevorzugte polymerisierbare organische Monomere sind kationisch polymerisierbare mono- oder polyfunktionelle Monomere, wie 3,4-Epoxycyclohexylmethyl-3,4-epoxycyclohexancarboxylat, Bis-(3,4-epoxycyclohexylmethyl)adipat, Vinylcyclohexendioxid, 3-Ethyl-3-hydroxymethyloxetan, 1,10-Decandiylbis(oxymethylen) bis (3-ethyloxetan) und 3,3-(4-Xylylendioxy)-bis-(methyl-3-ethyloxetan).

Als polymerisierbare Silane und Polysiloxane eigenen sich insbesondere solche Verbindungen, die radikalisch polymerisierbare Gruppen, vorzugsweise (Meth)acrylgruppen, oder kationisch polymerisierbare Gruppen, vorzugsweise Epoxid-, Oxetan-, Spiroorthoester oder Vinylethergruppen, aufweisen. Geeignete Silane und Polysiloxane sowie deren Herstellung werden in der DE 41 33 494 C2, DE 44 16 857 C1, EP 1 022 012 A2 und US 6,096,903 beschrieben. Bevorzugte Silane werden nachfolgendend aufgeführt. Besonders bevorzugt sind Polysiloxane auf der Basis dieser Silane, wobei die Polysiloxane in Form der Homo- und Cokondensate vorliegen können.

Bevorzugt sind Silane der allgemeinen Formel (1)

Y¹ ₙ₁Si X¹ ₘ₁R¹ ₄₋₍ₙ₁₊ₘ₁₎ (1)

in der die Reste X¹, Y¹ und R¹ gleich oder verschieden sind und folgende Bedeutung haben:
- R¹ =: Alkyl, Alkenyl, Aryl, Alkylaryl oder Arylalkyl,
- X¹ =: Wasserstoff, Halogen, Hydroxy, Alkoxy, Acyloxy, Alkylcarbonyl, Alkoxycarbonyl oder NR^{δ}₂ mit R^{δ} = Wasserstoff, Alkyl oder Aryl,
- Y¹ =: ein Substituent, der einen substituierten oder unsubstituierten 1,4,6-Trioxasprio-[4,4]-nonan-Rest enthält,
- n¹ =: 1,2 oder 3,
- m¹ =: 1,2 oder 3, mit n¹ + m¹ ≤ 4,
und Silane der allgemeinen Formel (2),

{X¹ ₙ₁R¹ ₖ₁Si[R²(A¹)₁]₄₋₍ₙ₁₊ₖ₁₎}ₓ₁B¹ (2)

in der Reste A¹, R¹, R² und X¹ gleich oder verschieden sind und folgende Bedeutungen haben:
- A¹ =: O, S, PR^{ε}, POR^{ε}, NHC(O)O oder NHC(O)ONR^{ε}, mit R^{ε} = Wasserstoff, Alkyl oder Aryl,
- B¹ =: ein geradkettiger oder verzweigter organischer Rest, der sich von einer Verbindung B¹' mit mindestens einer (für 1 = 1 und A = NHC(O)O oder NHC(O)NR^{ζ}) bzw. mindestens zwei C=C-Doppelbindungen und 5 bis 50 Kohlenstoffatomen ableitet, mit R^{ζ} = Wasserstoff, Alkyl oder Aryl,
- R¹ =: Alkyl, Alkenyl, Aryl, Alkylaryl oder Arylalkyl,
- R² =: Alkylen, Arylen oder Alkylenarylen,
- X¹ =: Wasserstoff, Halogen, Hydroxy, Alkoxy, Acyloxy, Alkylcarbonyl, Alkoxycarbonyl oder NR^{δ}₂, mit R^{δ} = Wasserstoff, Alkyl oder Aryl,
- n¹ =: 1, 2 oder 3,
- k¹ =: 0, 1 oder 2,
- l =: 0 oder 1,
- x¹ =: eine ganze Zahl, deren Maximalwert der Anzahl von Doppelbindungen in der Verbindung B¹' minus 1 entspricht, bzw. gleich der Anzahl von Doppelbindungen in der Verbindung B^{1,} ist, wenn 1 = 1 und A für NHC(O)O oder NHC(O)NR^{ε} steht.

Die Silane der allgemeinen Formel (1) und (2) sind hydrolysierbar und polymerisierbar, wobei die Reste X¹ hydrolysierbar und die Reste B¹ und Y¹ polymerisierbar sind und jeweils mindestens ein Rest B¹, X¹ und Y¹ mit der oben genannten Bedeutung in den Silanen der allgemeinen Formel (1) und (2) vorhanden ist. Polysiloxane auf der Basis der Silane (1) und/oder (2) sind bevorzugte polymerisierbare Komponenten.

Die Alkylreste der Verbindungen (1) und (2) sind z.B. geradkettige, verzweigte oder cyclische Reste mit 1 bis 20, vorzugsweise mit 1 bis 10 Kohlenstoffatomen, und besonders bevorzugt sind niedere Alkyl-Reste mit 1 bis 6 Kohlenstoffatomen. Spezielle Beispiele sind Methyl, Ethyl, N-Propyl, i-Propyl, n-Butyl, s-Butyl, t-Butyl, i-Butyl, n-Pentyl, n-Hexyl, Cyclohexyl, 2-Ethylhexyl, Dodecyl und Octadecyl.

Die Alkenylreste sind z.B. geradkettige, verzweigte oder cyclische Reste mit 2 bis 20, vorzugsweise mit 2 bis 10 Kohlenstoffatomen, und besonders bevorzugt sind niedere Alkenylreste mit 2 bis 6 Kohlenstoffatomen, wie z.B. Vinyl, Allyl oder 2-Butenyl.

Bevorzugte Arylreste sind Phenyl, Biphenyl und Naphthyl.

Die Alkoxy-, Acyloxy-, Alkylcarbonyl-, Alkoxycarbonyl- und Aminoreste leiten sich bevorzugt von den oben genannten Alkyl- und Arylresten ab. Spezielle Beispiele sind Methoxy, Ethoxy, n- und i-Propoxy, n-, i-, s- und t-Butoxy, Methylamino, Ethylamino, Dimethylamino, Diethylamino, N-Ethylanilino, Acetyloxy, Propionyloxy, Methylcarbonyl, Ethylcarbonyl, Methoxycarbonyl, Ethoxycarbonyl, Benzyl, 2-Phenylethyl und Tolyl.

Die genannten Reste können gegebenenfalls einen oder mehrere Substituenten tragen, z.B. Halogen, Alkyl, Hydroxyalkyl, Alkoxy, Aryl, Aryloxy, Alkylcarbonyl, Alkoxycarbonyl, Furfuryl, Tetrahydrofurfuryl, Amino, Alkylamino, Dialkylamino, Trialkylammonium, Amido, Hydroxy, Formyl, Carboxy, Mercapto, Cyano, Isocyanato, Nitro, Epoxy, SO₃H und PO₄H₂. Unter den Halogenen sind Fluor, Chlor und Brom bevorzugt.

Die substituierten bzw. unsubstituierten 1,4,6-Trioxaspiro-[4,4]-nonan-Gruppen sind über Alkylen- oder über Alkenylenreste, die durch Ether- oder Estergruppen unterbrochen sein können, an das Si-Atom gebunden.

Weiter bevorzugt sind Silane gemäß der Formel (3) sowie darauf basierende Polysiloxane 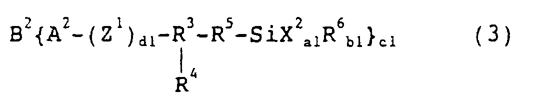 in der die Reste und Indices folgende Bedeutung haben:
- B² =: ein geradkettiger oder verzweigter organischer Rest mit mindestens einer C=C-Doppelbindung und 4 bis 50 Kohlenstoffatomen;
- X² =: Wasserstoff, Halogen, Hydroxy, Alkoxy, Acyloxy, Alkylcarbonyl, Alkoxycarbonyl oder NR⁷₂;
- R⁶ =: Alkyl, Alkenyl, Aryl, Alkylaryl oder Arylalkyl;
- R⁵ =: Alkylen, Arylen, Arylenalkylen oder Alkylenarylen mit jeweils 0 bis 10 Kohlenstoffatomen, wobei diese Reste durch Sauerstoff- und Schwefel-Atome oder durch Amino-Gruppen unterbrochen sein können;
- R⁷ =: Wasserstoff, Alkyl oder Aryl;
- A² =: O, S oder NH für d¹ = 1 und Z¹ = CO und R³ = Alkylen, Arylen oder Alkylenarylen mit jeweils 1 bis 10 Kohlenstoffatomen, wobei diese Reste durch Sauerstoff- und Schwefel-Atome oder durch Aminogruppen unterbrochen sein können, und R⁴ = COOH; oder
- A² =: O, S oder NH für d¹ = 1 und Z¹ = CO und R³ = Alkylen, Arylen oder Alkylenarylen mit jeweils 1 bis 10 Kohlenstoffatomen, wobei diese Reste durch Sauerstoff- und Schwefelatome oder durch Aminogruppen unterbrochen sein können, und R⁴ = H; oder
- A² =: O, S, NH oder COO für d¹ = 1 und Z¹ = CHR^{η}, mit R^{η} gleich H, Alkyl, Aryl oder Alkylaryl, und R³ = Alkylen, Arylen oder Alkylenarylen mit jeweils 1 bis 10 Kohlenstoffatomen, wobei diese Reste durch Sauerstoff- und Schwefelatome oder durch Aminogruppen unterbrochen sein können, und R⁴ = OH; oder
- A² =: O, S, NH oder COO für d¹ = 0 und R³ = Alkylen, Arylen oder Alkylenarylen mit jeweils 1 bis 10 Kohlenstoffatomen, wobei diese Reste durch Sauerstoff- und Schwefelatome oder durch Aminogruppen unterbrochen sein können, und R⁴ = OH; oder
- A² =: S für d¹ = 1 und Z² = CO und R³ = N und R⁴ = H;
- a¹ =: 1, 2 oder 3;
- b¹ =: 0, 1 oder 2;
- a¹+b¹ =: 3;
- c¹ =: 1, 2, 3 oder 4.

Die Silane der Formel (3) sind über die Reste B² polymerisierbar und über die Reste X² hydrolysierbar.

Die ggf. vorhandenen Alkyl-, Alkenyl-, Aryl-, Alkoxy-, Acyloxy-, Alkylcarbonyl-, Alkoxycarbonyl- und Aminoreste haben die für die Formeln (1) und (2) definierten Bedeutungen.

Für a¹ ≥ 2 bzw. b¹ = 2 können die Reste X² und R⁷ jeweils dieselbe oder eine unterschiedliche Bedeutung haben. Der Rest B² leitet sich ab von einer substituierten oder unsubstituierten Verbindung B²(A²H)_{c1} mit mindestens einer C=C-Doppelbindung, wie z.B. Vinyl-, Alkyl-, Acryl-, und/oder Methacrylgruppen, und 4 bis 50, vorzugsweise 6 bis 30 Kohlenstoffatomen. Vorzugsweise leitet sich B² ab von einer substituierten oder unsubstituierten Verbindung mit zwei oder mehreren Acrylat- oder Methacrylatgruppen. Derartige Verbindungen werden auch als (Meth)acrylate bezeichnet. Falls die Verbindung B²(A²H)_{c1} substituiert ist, können die Substituenten unter den oben genannten Substituenten ausgewählt sein. Die Gruppe - A²H kann - OH, -SH, -NH₂ oder -COOH sein und c kann Werte von 1 bis 4 einnehmen.

Besonders bevorzugt sind Silane gemäß der Formel (4) und darauf basierende Polysiloxane

[(W_{q}-R¹³-Z²)ₚ-R¹⁰]ₘ₂Y²-R⁹-SiX³ ₙ₂R⁸ ₃₋ₙ₂ Formel (4)

in der
- X³: für ein Halogenatom, eine Hydroxyl-, Alkoxy- und/oder Acyloxygruppe steht;
- n²: gleich 1 bis 3 ist;
- R⁸: für eine Alkyl-, Alkenyl-, Aryl-, Alkylaryl-, Arylalkylgruppe steht;
- R⁹: für eine Alkylengruppe steht;
- R¹⁰: für einen p-fach substituierten, geraden, verzweigten oder cyclischen, gesättigten oder ungesättigten, aromatischen oder aliphatischen organischen Rest mit 2 bis 40 Kohlenstoffatomen und ggf. 1 bis 6 Heteroatomen steht;
- R¹³: für einen q-fach substituierten, geraden, verzweigten oder cyclischen organischen Rest 1 bis 20 Kohlenstoffatomen steht oder entfällt;
- p: gleich 1 bis 2 ist;
- q: gleich 1 bis 6 ist;
- Y²: für -NR¹¹-, N oder -(C=O)-NH-steht;
- m²: gleich 2 für Y² = N und gleich 1 für Y = -NR¹¹- oder -(C=O)-NH-ist;
- R¹¹: für eine Alkyl- oder Arylgruppe steht;
- Z²: für O, S, -(C=O)-O-, -(C=O)-NH-, -O-(C=O)-NH- steht oder entfällt;
- W: für CH₂=CR¹²-(C=O)-O-steht; und
- R¹²: für ein Wasserstoffatom oder eine Alkylgruppe steht.

Geeignete Heteroatome sind Phosphor und vorzugsweise Sauerstoff.

Im Zusammenhang mit Formel (4) werden unter Alkyl-, Acyloxy-, Alkoxy-, Alkenylgruppen und Alkylengruppen Reste verstanden, die vorzugsweise 1 bis 25 Kohlenstoffatome, besonders bevorzugt 1 bis 10 Kohlenstoffatome und ganz besonders bevorzugt 1 bis 4 Kohlenstoffatome enthalten und gegebenenfalls einen oder mehrere Substituierten, wie zum Beispiel Halogenatome, Nitrogruppen oder Alkyloxyreste tragen. Mit Aryl sind Reste, Gruppen oder Substituenten gemeint, die vorzugsweise 6 bis 10 Kohlenstoffatome aufweisen und wie vorstehend angegeben substituiert sein können. Die obigen Definitionen gelten auch für zusammengesetzte Gruppen wie zum Beispiel Alkylaryl- und Arylalkylgruppen. Eine Alkylarylgruppe bezeichnet somit beispielsweise eine wie oben definierte Arylgruppe, die mit einer wie oben definierten Alkylgruppe substituiert ist.

Die Alkyl-, Acyloxy-, Alkoxy-, Alkenylgruppen und Alkylengruppen können geradkettig- verzweigt oder cyclisch sein.

Unabhängig voneinander wählbare bevorzugte Definitionen für die einzelnen Variablen der Formel (4) sind:
- X³ =: eine Methoxy- und/oder Ethoxygruppe;
- n² =: 2 oder 3;
- R⁸=: eine C₁ - bis C₃-Alkylgruppe, insbesondere eine Methylgruppe;
- R⁹=: eine C₁ - bis C₄-Alkylengruppe;
- R¹⁰ =: ein p-fach substituierter, gerader, verzweigter oder cyclischer, gesättigter oder ungesättigter, aromatischer oder aliphatischer organischer Rest mit 2 bis 10 Kohlenstoffatomen und ggf. einem Heteroatom, vorzugsweise einem Sauerstoffatom, besonders bevorzugt ein C₁ - bis C₄-Alkenylenrest oder ein monocyclischer Rest mit 4 bis 10, insbesondere 5 bis 8 Kohlenstoffatomen;
- R¹³=: ein q-fach substituierter, gerader, verzweigter oder cyclischer organischer Rest mit 1 bis 4 Kohlenstoffatomen, besonders bevorzugt und C₁ - bis C₃-Alkylenrest;
- p =: 1 oder 2, insbesondere 1;
- q =: 1 oder 2;
- Y² =: N oder -(C=O)-NH-;
- Z² =: -(C=O)-; und/oder
- R¹²=: ein Wasserstoffatom oder eine Methylgruppe.

Weiter bevorzugt sind hydrolysierbare und polymerisierbare Oxetansilane und deren Stereoisomeren entsprechen der allgemeinen Formel (5) sowie darauf basierende Polysiloxane: 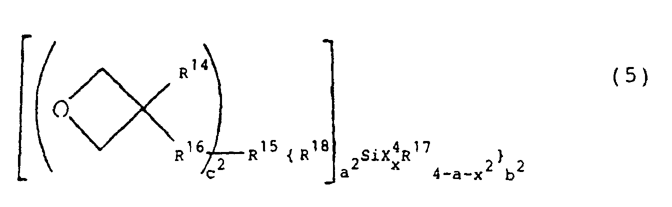 wobei die Variablen R¹⁴, R¹⁵, R¹⁶, R¹⁷, R¹⁸, R¹⁹, R²⁰, X⁴, Y³, a², b², c², und x², sofern nicht anders angegeben, unabhängig voneinander die folgenden Bedeutungen haben:
- R¹⁴ =: Wasserstoff oder substituiertes oder unsubstituiertes C₁- bis C₁₀-Alkyl;
- R¹⁵ =: entfällt oder substituiertes oder unsubstituiertes C₁- bis C₁₈-Alkylen, C₆- bis C₁₈-Arylen, C₇- bis C₁₈-Alkylenarylen oder -Arylenalkylen, wobei diese Reste durch mindestens eine Gruppe ausgewählt aus Ether-, Thioether-, Ester-, Carbonyl-, Amid- und Urethangruppe unterbrochen sein können;
- R¹⁶ =: entfällt oder substituiertes oder unsubstituiertes C₁- bis C₁₈-Alkylen, C₆- bis C₁₈-Arylen, C₇- bis C₁₈-Alkylenarylen oder C₇- bis C₁₈- Arylenalkylen, wobei diese Reste durch mindestens eine Gruppe ausgewählt aus Ether-, Thioether-, Ester-, Thioester-, Carbonyl-, Amid- und Urethangruppe unterbrochen sein oder diese endständig tragen können;
- R¹⁷ =: entfällt oder substituiertes oder unsubstituiertes C₁- bis C₁₈-Alkyl, C₂- bis C₁₈-Alkenyl, C₆- bis C₁₈-Aryl, C₇- bis C₁₈-Alkylaryl oder C₇- bis C₁₈-Arylalkyl, wobei diese Reste durch mindestens eine Gruppe ausgewählt aus Ether-, Thioether-, Ester-, Carbonyl-, Amid- und Urethangruppe unterbrochen sein können;
- R¹⁸ =: entfällt oder substituiertes oder unsubstituiertes -CHR²⁰-CHR²⁰-, -CHR²⁰-CHR²⁰-S-R¹⁹-, -S-R¹⁹-, -Y³-CO-NH-R¹⁹- oder -CO-O-R¹⁹-;
- R¹⁹ =: substituiertes oder unsubstituiertes C₁- bis C₁₈-Alkylen, C₆- bis C₁₈-Arylen, C₆- bis C₁₈-Alkylenarylen oder C₆- bis C₁₈-Arylenalkylen, wobei diese Reste durch mindestens eine Gruppe ausgewählt aus Ether-, Thioether-, Ester-, Carbonyl-, Amid- und Urethangruppe unterbrochen sein können;
- R²⁰ =: Wasserstoff oder substituiertes oder unsubstituiertes C₁- bis C₁₈-Alkyl oder C₆ bis C₁₀-Aryl;
- X⁴ =: eine hydrolysierbare Gruppe, nämlich Halogen, Hydroxy, Alkoxy oder Acyloxy;
- Y³ =: O oder S;
- a² =: 1, 2 oder 3;
- b² =: 1, 2 oder 3;
- c² =: 1 bis 6; und
- x² =: 1, 2 oder 3;
und mit der Maßgabe, daß (i) a²+x² = 2, 3 oder 4 und (ii) a² und/oder b² = 1.

Die obigen Formeln decken jedoch nur solche Verbindungen ab, die mit der Valenzlehre zu vereinbaren sind.

Üblicherweise liegen die Silane gemäß Formel (5) als Stereoisomeren-Gemische und insbesondere als Racemate vor.

Die bei den Resten der Formel (5) möglicherweise vorhandenen Ether-, Thioether-, Ester-, Thioester-, Carbonyl-, Amid- und Urethangruppen sind durch die folgenden Formeln definiert: -O-, -S-, -CO-O-, -O-CO-, -CO-S-, -S-CO-, -CS-O-, -O-CS-, -CO-, -CO-NH-, -NH-CO-, -O-CO-NH-und -NH-CO-O-.

Die in den Formeln (5) möglichen nicht-aromatischen Reste oder nicht-aromatischen Teile der Reste können geradkettig, verzweigt oder cyclisch sein.

In den Silanen gemäß Formel (5) ggf. vorhandenen Alkylreste haben bevorzugt 1 bis 8 und besonders bevorzugt 1 bis 4 Kohlenstoffatome. Spezielle Beispiele für mögliche Alkylreste sind Methyl, Ethyl, n- und iso-Propyl, sec.- und tert.-Butyl, n-Pentyl, Cyclohexyl, 2-Ethylhexyl und Octadecyl.

In den Silanen gemäß Formel (5) ggf. vorhandenen Alkenylreste haben bevorzugt 2 bis 10 und besonders bevorzugt 2 bis 6 Kohlenstoffatome. Spezielle Beispiele für mögliche Alkenylreste sind Vinyl, Allyl- und iso-Butenyl.

Bevorzugte Beispiel für mögliche Aryl-Reste der Formel (5) sind Phenyl, Biphenyl und Naphthyl. Alkoxyreste haben bevorzugt 1 bis 6 Kohlenstoffatome. Spezielle Beispiel für mögliche Alkoxyreste sind Methoxy, Ethoxy, n- Propoxy, iso-Propoxy und tert.-Butoxy. Acyloxyreste haben vorzugsweise 2 bis 5 Kohlenstoffatome. Spezielle Beispiele sind Acetyloxy und Propionyloxy.

Bevorzugte Alkylenreste der Formel (5) leiten sich von den obigen bevorzugten Alkylresten ab, und bevorzugte Arylenreste leiten sich von den obigen bevorzugten Arylresten ab. Bevorzugte aus einer Kombination von nicht-aromatischem und aromatischem Teil bestehende Reste, wie Alkylaryl-, Arylalkyl-, Alkylenarylen- und Arylenalkylenreste, leiten sich von den obigen bevorzugten Alkyl- und Arylresten ab. Spezielle Beispiele hierfür sind Benzyl, 2-Phenylethyl und Tolyl.

Die genannten substituierten R-Reste der Formel (5) tragen einen oder mehrere einfache Substituenten. Beispiele für diese Substituenten sind Methyl, Ethyl, Phenyl, Benzyl, Hydroxymethyl, Hydroxyethyl, Methoxy, Ethoxy, Chlor, Brom, Hydroxy, Mercapto, Isocyanato, Vinyloxy-, Acryloxy-, Methacryloxy-, Allyl-, Styryl, Epoxy, Carboxyl, SO₃H, PO₃H₂ oder PO₄H₂.

Für a², b², c² oder x² ≥ 2 können die Reste X⁴ sowie die einzelnen R-Reste jeweils dieselbe oder eine unterschiedliche Bedeutung haben.

Außerdem existieren für die oben angegebenen Variablen der Formel (5) bevorzugte Definitionen, die, sofern nicht anders angegeben, unabhängig voneinander gewählt werden können und wie folgt sind:
- R¹⁴ =: Wasserstoff oder C₁- bis C₅-Alkyl;
- R¹⁵ =: C₁- bis C₈-Alkylen, wobei diese Reste durch mindestens eine Gruppe ausgewählt aus Ether-, Thioether-, Ester- und Urethangruppe unterbrochen sein können;
- R¹⁶ =: entfällt oder C₁- bis C₈-Alkylen, wobei diese Reste durch mindestens eine Gruppe ausgewählt aus Ether-, Thioether-, Ester-, Thioester, Carbonyl-, Amid- und Urethangruppe unterbrochen sein oder diese endständig tragen können;
- R¹⁷ =: entfällt oder Methyl, Ethyl oder Phenyl;
- R¹⁸ =: entfällt oder -CHR²⁰-CHR²⁰-, -S-R¹⁹-, -Y-CO-NH-R¹⁹- oder - CO-O-R¹⁹-;
- R¹⁹ =: C₁- bis C₈-Alkylen, wobei diese Reste durch mindestens eine Gruppe ausgewählt aus Ether-, Thioether-, Ester-, Carbonyl-, Amid- und Urethangruppe unterbrochen sein können;
- R²⁰ =: Wasserstoff oder C₁- bis C₅-Alkyl;
- X⁴ =: Methoxy, Ethoxy oder Chlor;
- Y³ =: O oder S;
- a² =: 1;
- b² =: 1;
- c² =: 1 bis 6;
- x² =: 2 oder 3; und/oder
- a²+x²=: 3.

Dabei können die einzelnen R-Reste wiederum einfache Substituenten tragen.

Bevorzugte Verbindungen gemäß Formel (5) sind demgemäß solche, bei denen mindestens eine der Variablen der Formel (5) die vorstehend beschriebene bevorzugte Definition aufweist.

Weiter sind solche Oxetansilane der Formel (5) bevorzugt, bei denen die Indices a², b² und/oder c² den Wert 1 haben.

Die Silane (5) sind über die Oxetan-Gruppen polymerisierbar und über die Reste X⁴ hydrolysierbar.

Die oben genannten Silane können entweder allein oder zusammen mit anderen hydrolytisch kondensierbaren Verbindungen des Siliciums, Aluminiums, Zirkoniums, Titans, Bors, Zinns, Vanadiums und/oder Phosphors zu den Polysiloxanen verarbeitet werden. Diese zusätzlichen Verbindungen können entweder als solche oder bereits in vorkondensierter Form eingesetzt werden.

Bevorzugte weitere hydrolytisch kondensierbare Verbindungen des Siliciums sind Silane der allgemeinen Formel (6)

R²¹ ₖ₂(Z³R²²)ₘ₃SiX⁵ ₄₋₍ₖ₂₊ₘ₃₎ Formel (6)

in der
- R²¹: für eine C₁- bis C₈-Alkyl-, C₂- bis C₁₂-Alkenyl- oder C₆- bis C₁₄-Arylgruppe steht;
- R²²: für eine C₁- bis C₈-Alkylen-, C₂- bis C₁₂-Alkenylen- oder C₆- bis C₁₄-Arylengruppe steht;
- X⁵: für ein Wasserstoff- oder Halogenatom oder eine C₁- bis C₈- Alkoxygruppe steht;
- Z³: für eine Glycidyl-, Acryl-, Methacryl-, Vinyl-, Allyl- oder Vinylethergruppe steht;
- k²: gleich 0, 1, 2 oder 3 ist;
- m³: gleich 0, 1, 2 oder 3 ist; und
- k²+m³: gleich 0, 1, 2 oder 3 ist.

Unabhängig voneinander wählbare bevorzugte Definitionen für die einzelnen Variablen sind:
- R²¹ =: eine C₁- bis C₃-Alkyl-, C₂- bis C₅-Alkenyl- oder eine Phenylgruppe;
- R²² =: eine C₁- bis C₅-Alkylen-, C₂- bis C₅-Alkenylen- oder eine Phenylengruppe;
- X⁵ =: ein Halogenatom, eine Methoxy- oder Ethoxygruppe;
- Z³ =: eine Acryl- oder Methacrylgruppe;
- k² =: 0 und 1;
- m³ =: 0 und 1;
- k²+m³ =: 0, 1 oder 2.

Derartige Silane sind beispielsweise in der DE 34 07 087 A1 beschrieben.

Bevorzugte Zirkonium-, Titanverbindungen zur Cokondensation mit den genannten Silanen sind solche gemäß Formel (7)

MeX⁶ _{y}R²³ _{z} Formel (7)

in der
- Me: für Zr oder Ti steht;
- R²³: für ein Wasserstoffatom, eine substituierte oder unsubstituierte C₁- bis C₁₂-Alkyl-, C₁- bis C₁₅-Alkylaryl- oder C₆- bis C₁₄-Arylgruppe steht;
- X⁶: für ein Halogenatom, eine Hydroxyl- oder C₁-bis C₈-Alkoxygruppe steht;
- y: gleich 1 bis 4 ist;
- z: gleich 0 bis 3 ist.

Unabhängig voneinander wählbare bevorzugte Definitionen für die einzelnen Variablen sind:
- R²³ =: eine C₁- bis C₅-Alkyl- oder eine Phenylgruppe;
- X⁶ =: ein Halogenatom, eine Methoxy-, Ethoxy- oder Propoxygruppe;
- y =: 4;
- z =: 0 oder 1, insbesondere 0.

Besonders bevorzugte Zirkonium- und Titanverbindungen sind ZrCl₄, Zr(OC₂H₅)₄, Zr(OC₃H₇)₄, Zr(OC₄H₉)₄, ZrOCl₂, TiCl₄, Ti(OC₂H₅)₄, Ti(OC₃H₇)₄ und Ti(OC₄H₉)₄.

Bevorzugte Aluminiumverbindungen sind solche gemäß der Formel (8)

AlR²⁴ ₃ Formel (8)

in der
- R²⁴: für ein Halogenatom, eine Hydroxyl- oder C₁- bis C₈-Alkoxygruppe, vorzugsweise für ein Halogenatom oder eine C₁- bis C₅-Alkoxygruppe steht.

Besonders bevorzugte Aluminiumverbindungen sind Al(OCH₃)₃, Al(OC₂H₅)₃, Al(OC₃H₇)₃, Al(OC₄H₉)₃ und AlCl₃.

Darüber hinaus eignen sich Bortrihalogenide, Zinntetrahalogenide, Zinntetraalkoxide und/oder Vanadylverbindungen zur Cokondensation mit den oben genannte Silanen.

Die Aushärtung der Materialien erfolgt in Abhängigkeit von dem verwendeten Initiator durch thermische, photochemische oder redoxinduzierte Polymerisation.

Als Initiatoren für die heißhärtenden Systeme sind Peroxide, insbesondere Dibenzoylperoxid, Dilauroylperoxid, tert.-Butylperoctat und tert.-Butylperbenzoat bevorzugt. Darüber hinaus sind auch Azobisisobutyroethylester, 2,2'-Azoisobuttersäurenitril (AIBN), Benzpinakol und 2,2'-Dialkylbenzpinakole geeignet.

Als Initiatoren für die Kaltpolymerisation werden Radikale liefernde Systeme, zum Beispiel Benzoylperoxid, Lauroylperoxid oder vorzugsweise Dibenzoylperoxid, zusammen mit Aminen wie N,N-Dimethyl-p-toluidin, N,N-Dihydroxyethyl-p-toluidin, N,N-Dimethylsym.-xylidin oder anderen strukturverwandten Aminen eingesetzt. Amin und Peroxyd werden üblicherweise auf zwei unterschiedliche Komponenten des Dentalwerkstoffs verteilt. Beim Mischen der aminhaltigen Basenpaste mit der peroxidhaltigen Initiatorpaste wird durch die Reaktion von Amin und Peroxid die radikalische Polymerisation initiiert.

Als Initiatoren für die Photopolymerisation können zum Beispiel Benzophenon und seine Derivate sowie Benzoin und seine Derivate verwendet werden. Weitere bevorzugte Photoinitiatoren sind die α-Diketone wie 9,10-Phenanthrenchinon, Diacetyl, Furil, Anisil, 4,4'-Dichlorbenzil und 4,4'-Dialkoxybenzil. Besonders bevorzugt wird Campherchinon und 2,2-Methoxy-2-phenyl-acetophenon und insbesondere α-Diketone in Kombination mit Aminen als Reduktionsmittel verwendet. Bevorzugte Amine sind 4-(N,N-Dimethylamino)-benzoesäureester, N,N-Dimethylaminoethylmethacrylat, N,N-Dimethyl-sym.-xylidin und Triethanolamin. Darüber hinaus sind auch Acylphosphine, wie z.B. 2,4,6-Trimethylbenzoyldiphenyloder Bis-(2,6-dichlorbenzoyl)-4-N-propylphenylphosphinoxid als Photoinitiatoren besonders geeignet.

Für die Härtung von kationisch polymerisierbaren Systemen eignen sich besonders Diaryliodonium- oder Triarylsulfoniumsalze, wie z.B. Triphenylsulfoniumhexafluorophosphat oder -hexafluoroantimonat sowie die in der WO 96/13538 und WO 98/47047 beschriebenen Photoinitiatorsysteme.

Weiterhin können die Mischungen zur Verbesserung der mechanischen Eigenschaften mit organischen oder anorganischen Partikeln oder Fasern gefüllt sein. Als Füllstoffkomponenten eignen sich insbesondere amorphe, kugelförmige Materialien auf der Basis von Mischoxiden aus SiO₂, ZrO₂ und/oder TiO₂ mit einer mittleren durchschnittlichen Partikelgröße von 0,005 bis 2,0 µm, vorzugsweise von 0,1 bis 1 µm, wie sie beispielsweise in der DE-PS 32 47 800 offenbart werden, mikrofeine Füllstoffe, wie pyrogene Kieselsäure oder Fällungskieselsäure sowie Makro- oder Mini-Füllstoffe, wie Quarz-, Glaskeramik- oder Glaspulver mit einer durchschnittlichen Teilchengröße von 0,01 bis 20 µm, vorzugsweise 0,5 bis 5 µm, sowie röntgenopake Füllstoffe, wie Ytterbiumfluorid. Unter Mini-Füllstoffen werden Füllstoffe mit einer Partikelgröße von 0,5 bis 1,5 µm und unter Makro-Füllstoffen Füllstoffe mit einer Partikelgröße von 10 bis 20 µm verstanden.

Bevorzugte erfindungsgemäße Zusammensetzungen enthalten bezogen auf ihre Gesamtmasse:
(a) 5 bis 90 Gew.-%, insbesondere 5 bis 40 Gew.-%, ganz besonders bevorzugt 10 bis 20 Gew.-% eines oder mehrerer Cluster gemäß Formel (I),
(b) 10 bis 90 Gew.-%, insbesondere 10 bis 80 Gew.-% einer oder mehrerer weiterer polymerisierbarer Komponenten,
(c) 0,1 bis 5,0 Gew.-%, insbesondere 0,2 bis 2,0 Polymerisationsinitiator,
   und
(d) 0 bis 90 Gew.-%, insbesondere 0 bis 80 Gew.-% Füllstoff.

Die obige Zusammensetzung kann in Abhängigkeit vom gewünschten Anwendungszweck weiter optimiert werden. So enthält ein Material, das sich besonders als dentales Füllungsmaterial eignet, jeweils bezogen auf die Gesamtmasse des Materials vorzugsweise:
(a) 5 bis 20 Gew.-% eines oder mehrerer Cluster gemäß Formel (I),
(b) 0 bis 20 Gew.-% einer oder mehrerer weiterer polymerisierbarer Komponenten,
(c) 0,2 bis 2,0 Gew.-% 2,0 Polymerisationsinitiator,
   und
(d) 5 bis 80 Gew.-% Füllstoff.

Ein Dentalmaterial, das sich besonders als dentaler Zement eignet, enthält, jeweils bezogen auf die Gesamtmasse des Materials, vorzugsweise:
(a) 5 bis 30 Gew.-% eines oder mehrerer Cluster gemäß Formel (I),
(b) 0 bis 30 Gew.-% einer oder mehrerer weiterer polymerisierbarer Komponenten,
(c) 0,2 bis 2,0 Gew.-% 2,0 Polymerisationsinitiator,
   und
(d) 5 bis 60 Gew.-% Füllstoff.

Ein Dentalmaterial, das sich besonders als dentales Beschichtungsmaterial eignet, enthält, jeweils bezogen auf die Gesamtmasse des Materials, vorzugsweise:
(a) 5 bis 40 Gew.-% eines oder mehrerer Cluster gemäß Formel (I),
(b) 5 bis 80 Gew.-% einer oder mehrerer weiterer polymerisierbarer Komponenten,
(c) 0,2 bis 2,0 Gew.-% 2,0 Polymerisationsinitiator,
   und
(d) 0 bis 40 Gew.-% Füllstoff.

Ganz besonders bevorzugt sind Materialien, die als weitere polymerisierbare Komponente (b) 10 bis 90 Gew.-% Polysiloxan und 0 bis 40 Gew.-% polymerisierbare organische Monomere enthalten, jeweils bezogen auf die Gesamtmasse des Dentalmaterials.

Diese Zusammensetzungen eignen sich besonders als Dentalmaterialien, ganz besonders als Adhäsive, beispielsweise für Inlays, Beschichtungsmaterialien, Zemente und insbesondere Füllungsmaterialien. Generell sind die Zusammensetzungen besonders für solche Anwendungen geeignet bei denen die Aushärtung des Materials in der Mundhöhle erfolgt.

Die erfindungsgemäßen Dentalmaterialien weisen nach der Polymerisation nur einen minimalen Gehalt an mit wäßrigen oder alkoholischen Lösungsmitteln herauslösbaren unpolymerisierten Bestandteilen auf, was eine deutliche Verbesserung gegenüber herkömmlichen Dentalwerkstoffen darstellt, da toxische Nebenwirkungen durch monomere Bestandteile unterdrückt werden.

Erfindungsgemäß werden zur Herstellung von Dentalmaterialien Cluster mit definierter Größe und Gestalt, d.h. reine, definierte Verbindungen mit bekannter Stöchiometrie eingesetzt, besonders bevorzugt Cluster mit monodisperser Massenverteilung. Auf diese Weise lassen sich die Materialeigenschaften der Dentalwerkstoffe, wie beispielsweise E-Modul, Festigkeit, Härte und Abrasivität, kontrolliert einstellen und verbessern. Dentalmaterialien die 1 bis 2 unterschiedliche Cluster enthalten sind bevorzugt.

Im folgenden wird die Erfindung anhand von Beispielen näher erläutert

### Beispiel 1:

### Synthese des Oxozirkonium-Methacrylat-Clusters der Zusammensetzung Zr₄O₂(OMc)₁₂

Zu 1,73 g (3,6 mmol) einer 80 %-igen Lösung von Zirkoniumbutylat (Zr(OC₄H₉)₄) in n-Butanol wurden 2,04 g (24 mmol) Methacrylsäure gegeben. Die Reaktionsmischung wurde für einen Tag bei Raumtemperatur stehengelassen und der gebildete Niederschlag unter Feuchtigkeitsausschluß abfiltriert. Es ergaben sich 1,09 g (86% Ausbeute) an farblosen kubischen Kristallen die in Chloroform, Ethanol oder Toluol löslich sind.

### Beispiel 2:

### Synthese von Matrixstoffen auf Kieselsäurepolykondensat-Basis

### A) Hydrolytische Kondensation von Bis[(methacryloyloxy)propoxycarbonylethyl)-[3-(triethoxysilylpropyl)]amin:

16,1 g (26 mmol) Bis[(methacryloyloxy)propoxycarbonylethyl)-[3-(triethoxysilylpropyl)]amin, das durch Michael-Addition von 3-Aminopropyltriethoxysilan an 2-(Acryloyloxyethyl)-propylmethacrylat zugänglich ist (vgl. EP 1 022 012), wurden in 37,5 ml wasserfreiem Ethanol gelöst und unter Zugabe von 2,81 g einer wäßrigen 0,1 N Ammoniumfluoridlösung hydrolytisch kondensiert. Nach 24 h Rühren bei Raumtemperatur wurden die flüchtigen Komponenten im Vakuum entfernt und es blieben ca. 12 g eines relativ niedrigviskosen Harzes (SG-1) mit einer Viskosität von η = ca. 8 Pas (23°C) zurück. Bei dieser und allen anderen Viskositätsangaben handelt es sich, wenn nicht anders angegeben, um die Rotationsviskosität gemessen mit einem Rotationsrheometer mit Platte-Platte-Meßsystem, CV = 120 (Modell CVO 120 der Firma Bohlin).

### B) Hydrolytische Kondensation von (3-Triethoxysilylpropylaminocarbonyl)buttersäure-(1,3(2)-bismethacryloyloxypropyl)ester:

10,9 g (20 mmol) (3-Triethoxysilylpropylamido)buttersäure-(1,3-(2)-bismethacryloyloxypropyl)ester, der durch Amidbildung aus 3-Aminopropyltriethoxysilan mit dem Addukt aus Glycerindimethacrylat und Glutarsäureanhydrid erhalten wurde (vgl. EP 1 022 012), wurden in 98,2 ml wasserfreiem Ethylacetat gelöst und unter Zugabe von 1,08 g 0,5 N Salzsäure hydrolytisch kondensiert. Nach 30 Minuten Rühren bei 40°C wurden die flüchtigen Komponenten im Vakuum entfernt. Anschließend wurde das erhaltende Harz nach Lösen in einer Mischung aus 35 g tert.-Butylmethyl-ether, 12 g THF und 1,45 g (12 mmol) 2,4,6-Trimethylpyridin durch tropfenweise Zugabe von 1,96 g (18 mmol) Trimethylchlorsilan silyliert. Nach Rühren über Nacht bei Raumtemperatur wurde die Reaktionsmischung mit verdünnter Salzsäure und gesättigter NaCl-Lösung gewaschen und dann über wasserfreiem Natriumsulfat getrocknet. Nach dem Abdampfen des Lösungsmittels im Vakuum verblieben ca. 6 g eines viskosen Harzes (SG-2) mit einer Viskosität von η = ca. 75 Pas (23°C).

### Beispiel 3:

### Herstellung von Dentalmaterialien auf der Basis von Clusteren gemäß Beispiel 1

Ausgehend von dem Cluster Zr₄O₂(OMc)₁₂ aus Beispiel 1 und den Matrixstoffen SG-1 und SG-2 wurden verschiedene Materialien hergestellt. Dabei wurden die Cluster als 10 %-ige Lösung in Ethanol mit den Matrixstoffen gemischt und nach Zugabe der Initiatorkomponenten das Lösungsmittel im Vakuum abgedampft. Die Zusammensetzungen (Masse-%) der so hergestellten ungefüllten Materialien M-1 bis M-5 sind in Tabelle 1 aufgeführt. Zur Bestimmung der mechanischen Eigenschaften wurden aus den Zusammensetzungen Prüfkörper mit den Abmessungen 25 x 2 x 2 mm hergestellt und diese durch Belichten mit Licht einer Wellenlänge von 390 bis 500 nm (6 Minuten) ausgehärtet. Hierzu wurde eine dentale Strahlungsquelle des Typs Spectramat der Firma Vivadent verwendet. Die Biegefestigkeit (BF) bzw. der Biege-E-Modul (BEM) wurden nach der ISO-Norm 4049 (2000) bestimmt, wobei die Prüfkörper vorher bei 37°C für 24h in Wasser gelagert wurden. Darüber hinaus wurden BF- und BEM-Werte auch von Proben gemessen, die für 24 h bei 37°C trocken gelagert wurden.

**Tabelle 1:**

| Zusammensetzung (Masse-%) der ungefüllten Materialien M-1 bis M-5 | | | | | |
|---|---|---|---|---|---|
| | **M-1*)** | **M-2** | **M-3*)** | **M-4** | **M-5** |
| **SG-1** | 98,7 | 88,7 | - | - | - |
| **SG-2** | - | - | 98,7 | 88,7 | 78,7 |
| **Zr-Cluster aus Beispiel 1** | - | 10,0 | - | 10,0 | 20 |
| **Photoinitiator**^{**a)**} | 1,3 | 1,3 | 1,3 | 1,3 | 1,3 |

| | | | | | |
|---|---|---|---|---|---|
| *) Vergleichsbeispiel ohne Cluster | | | | | |
| ^{a)} als Photoinitiator wurde eine Mischung aus 0,3 % Campherchinon, 0,6 % 4-(N,N-Dimethylamino)-benzoesäureethylester und 0,4 % Acylphosphinoxid (Lucerin TPO, BASF) verwendet | | | | | |

**Tabelle 2:**

| Biegefestigkeit (BF) und Biege-E-Moduls (BEM) der Materialien M-1 bis M-5 | | | | | |
|---|---|---|---|---|---|
| | **M-1*)** | **M-2** | **M-3*)** | **M-4** | **M-5** |
| **BF trocken (MPa)** | 46 | 47 | 48 | 59 | 70 |
| **BF H**_{**2**}**O-Lagerung (MPa)** | 31 | 52 | 36 | 60 | 60 |
| **BEM trocken (MPa)** | 1230 | 1900 | 1820 | 2100 | 2400 |
| **BEM H**_{**2**}**O-Lagerung (MPa)** | 1000 | 1920 | 1750 | 2270 | 2600 |

| | | | | | |
|---|---|---|---|---|---|
| *) Vergleichsbeispiel ohne Cluster | | | | | |

Zur Herstellung der Kompositpasten K-1 bis K-5 wurden die ungefüllten Materialien M-1 bis M-5 mit den in Tabelle 3 angegebenen Mengen an Füllstoff gemischt. Als Füllstoffe wurden silanisierte pyrogenen Kieselsäure mit einer mittleren Primärteilchengröße von 40 nm und einer BET-Oberfläche von 50 m²/g (silanisiertes Aerosil OX-50, Degussa), Ytterbiumtrifluorid mit einer mittleren Korngröße von 5 µm und einer BET-Oberfläche von < 7,5 m²/g (YbF₃, Auer Remy), silanisiertes SiO₂-ZrO₂-Mischoxid mit einer Primärteilchengröße von 130 bis 230 nm (Sphärosil, Tokoyma Soda) und silanisiertes Bariumsilikatglas (Ba-Si-Glas) mit einer mittleren Partikelgröße von 1,2 µm verwendet. Die Füllstoffkomponenten werden mittels eines Kapselvibrators eingearbeitet.

Anschließend wurden die Biegefestigkeit und der Biege-E-Modul gemessen, jeweils nach 24stündiger Lagerung in Wasser oder im Trockenen. Die Ergebnisse sind in Tabelle 4 zusammengefaßt.

**Tabelle 3:**

| Zusammensetzung der Kompositpasten K-1 bis K5 (Angaben in Masse-%) | | | | | |
|---|---|---|---|---|---|
| | **K-1*)** | **K-2** | **K-3*)** | **K-4** | **K-5** |
| **ungefülltes** | 25,0 | 25,0 | 25,0 | 25,0 | 25,0 |
| **Material** | (M-1) | (M-2) | (M-3) | (M-4) | (M-5) |
| **Aerosil OX-50** | 1,0 | 1,0 | 1,0 | 1,0 | 1,0 |
| **YbF**_{**3**} | 15,0 | 15,0 | 15,0 | 15,0 | 15,0 |
| **Sphärosil** | 15,0 | 15,0 | 15,0 | 15,0 | 15,0 |
| **Ba-Si-Glas** | 44,0 | 44,0 | 44,0 | 44,0 | 44,0 |

| | | | | | |
|---|---|---|---|---|---|
| *) Vergleichsbeispiel ohne Cluster | | | | | |

**Tabelle 4:**

| Biegefestigkeit (BF) und Biege-E-Moduls (BEM) der Komposite K-1 bis K-5 | | | | | |
|---|---|---|---|---|---|
| | **K-1*)** | **K-2** | **K-3*)** | **K-4** | **K-5** |
| **BF trocken (MPa)** | 94 | 96 | 89 | 101 | 103 |
| **BF H**_{**2**}**O-Lagerung (MPa)** | 68 | 100 | 89 | 110 | 113 |
| **BEM trocken (MPa)** | 6400 | 8900 | 8150 | 9100 | 9840 |
| **BEM H**_{**2**}**O-Lagerung (MPa)** | 5500 | 9300 | 7160 | 9750 | 10900 |

| | | | | | |
|---|---|---|---|---|---|
| *) Vergleichsbeispiel ohne Cluster | | | | | |

Die Ergebnisse zeigen (Tabellen 2 und 4), daß die Zugabe von Clustern der Zusammensetzung Zr₄O₂(OMc)₁₂ jeweils zu einer Verbesserung der Festigkeit und zu einer Erhöhung des E-Moduls Materialien führt. Darüber hinaus läßt sich bei den clusterhaltigen Materialien auch eine Erhöhung des E-Moduls nach Wasserlagerung beobachten, während der E-Modul bei den nichtmodifizierten Proben M-1/K-1 und M-3/K-3 nach Wasserlagerung abnimmt.

Proben der ausgehärteten Komposite K-2 und K-4 wurden zerstoßen und die Bruchstücke in Ethanol bei 37°C dispergiert. Nach 72 h wurden die festen Bestandteile abfiltriert und das Filtrat zur Trockene eingeengt. Es ergab sich praktisch kein Rückstand, was auf eine vollständige Einbindung der polymerisationsfähigen Komponenten in das Polymernetzwerk der Kompositmatrix hinweist.

## Patentansprüche

1. Dentalmaterial enthaltend einen Cluster gemäß der allgemeinen Formel
[(M¹)ₐ(M²)_{b}O_{c}(OH)_{d}(OR)ₑ(L-Sp-Z)_{f}] (I)
in der
M¹, M² unabhängig voneinander jeweils für ein Metallatom der III. oder V. Hauptgruppe oder der I. bis VIII. Nebengruppe des Periodensystems der Elemente stehen;
R eine Alkylgruppe mit 1 bis 6 Kohlenstoffatomen ist;
L eine koordinierende Gruppe mit 2 bis 6 komplexierenden Zentren ist;
Sp eine Spacergruppe ist oder entfällt;
Z eine polymerisationsfähige Gruppe ist;
a eine Zahl von 2 bis 20 ist;
b eine Zahl von 0 bis 10 ist;
c eine Zahl von 1 bis 30 ist;
d, e unabhängig voneinander jeweils eine Zahl von 0 bis 30 sind;
f eine Zahl von 2 bis 30 ist,
wobei eine eventuell vorhandene Ladung des Clusters (I) durch Gegenionen ausgeglichen wird.

2. Dentalmaterial nach Anspruch 1, **dadurch gekennzeichnet,** da**ß** die Variablen die folgenden Bedeutungen haben:
M¹, M² = unabhängig voneinander Ti und/oder Zr;
R = eine Alkylgruppe mit 1 bis 4 Kohlenstoffatomen, insbesondere 1 bis 2 Kohlenstoffatomen;
L = α-Hydroxycarboxylat (-CH(OH)-COO⁻), α-Aminocarboxylat (-CH(NH₂)-COO⁻), β-Diketonat ([-C(-O⁻)=CH-C(=O)R^{κ}]; mit R^{κ} = Alkyl, vorzugsweise C₁- bis C₆-Alkyl, besonders bevorzugt C₁- bis C₃-Alkyl, insbesondere Methyl, Sulfonat (-SO₃⁻) oder Phosphonat (-PO₃²⁻), besonders bevorzugt Carboxylat (-COO⁻);
Sp = eine Alkylengruppe mit 1 bis 18 Kohlenstoffatomen, eine Oxyalkylengruppe 1 bis 18 Kohlenstoffatomen und 0 bis 6 Sauerstoffatomen oder eine Arylengruppe mit 6 bis 14 Kohlenstoffatomen, wobei der Spacer Sp eine oder mehrere, vorzugsweise 0 bis 2 der Gruppen O, S, CO-O, O-CO, CO-NH, NH-CO, O-CO-NH, NH-CO-O, und NH enthalten kann; besonders bevorzugt eine Alkylengruppe mit 1 bis 6, insbesondere 1 bis 3 Kohlenstoffatomen oder entfällt;
Z = eine ethylenisch ungesättigte Gruppe, eine Epoxid-, Oxetan-, Vinylether-, 1,3-Dioxolan-, Spiroorthoester-, besonders bevorzugt eine Methacryl- und/oder Acrylgruppe;
a = 2 bis 11;
b = 0 bis 4.

3. Dentalmaterial nach Anspruch 2, **dadurch gekennzeichnet,** da**ß** L-Sp-Z für Acrylat, Methacrylat, Oleat, Allylacetoacetat und/oder Acetoacetoxyethylmethacrylat steht.

4. Dentalmaterial nach einem der Ansprüche 2 bis 3, **dadurch gekennzeichnet, daß** der Cluster 1 bis 4 Arten von Liganden des Typs L-Sp-Z enthält.

5. Dentalmaterial nach einem der Ansprüche 2 bis 4, **dadurch gekennzeichnet, daß** der Cluster eine monodisperse Massenverteilung aufweist.

6. Dentalmaterial nach Anspruch 2 oder 5, **dadurch gekennzeichnet, daß** die Indices c bis f solche Werte annehmen, daß die positiven Ladungen des oder der Metalle vollständig ausgeglichen werden.

7. Dentalmaterial nach einem der Ansprüche 2 bis 6, **dadurch gekennzeichnet, daß** M¹ gleich M² ist.

8. Dentalmaterial nach einem der Ansprüche 2 bis 7, **dadurch gekennzeichnet, daß** es eine oder mehrere weiteren polymerisierbaren Komponenten enthält.

9. Dentalmaterial nach Anspruch 8, **dadurch gekennzeichnet, daß** die weitere polymerisierbare Komponente ein polymerisierbares Polysiloxan, ein ionisch und/oder radikalisch polymerisierbares organisches Monomer oder eine Mischung davon ist.

10. Dentalmaterial nach einem der Ansprüche 2 bis 9, **dadurch gekennzeichnet, daß** es einem Initiator für die ionische und/oder radikalische Polymerisation, Füllstoff und/oder weitere Additiven enthält.

11. Dentalmaterial nach einem der vorangehenden Ansprüche, **dadurch gekennzeichnet, daß** es bezogen auf seine Gesamtmasse
(a) 5 bis 90 Gew.-% mindestens eines Clusters gemäß Formel (I),
(b) 10 bis 90 Gew.-% weitere polymerisierbare Komponente,
(c) 0,1 bis 5,0 Gew.-% Polymerisationsinitiator,
und
(d) 0 bis 90 Gew.-% Füllstoff enthält.

12. Verwendung eines Clusters der allgemeinen Formel
[(M¹)ₐ(M²)_{b}O_{c}(OH)_{d}(OR)ₑ(L-Sp-Z)_{f}] (I)
in der
M¹, M² unabhängig voneinander jeweils für ein Metallatom der III. oder V. Hauptgruppe oder der I. bis VIII. Nebengruppe des Periodensystems der Elemente stehen;
R eine Alkylgruppe mit 1 bis 6 Kohlenstoffatomen ist;
L eine koordinierende Gruppe mit 2 bis 6 komplexierenden Zentren ist;
Sp eine Spacergruppe ist oder entfällt;
Z eine polymerisationsfähige Gruppe ist;
a eine Zahl von 2 bis 20 ist;
b eine Zahl von 0 bis 10 ist;
c eine Zahl von 1 bis 30 ist;
d, e unabhängig voneinander jeweils eine Zahl von 0 bis 30 sind;
f eine Zahl von 2 bis 30 ist,
wobei eine eventuell vorhandene Ladung des Clusters (I) durch Gegenionen ausgeglichen wird, als Dentalmaterial oder zur Herstellung eines Dentalmaterials.

13. Verwendung nach Anspruch 12 als Adhäsiv, Beschichtungsmaterial, Zement oder Füllungsmaterial.

## Claims

1. Dental material comprising a cluster according to the general formula
[(M¹)ₐ(M²)_{b}O_{c}(OH)_{d}(OR)ₑ(L-Sp-Z)ᵣ] (I)
in which
M¹, M² independently of each other, stand for a metal atom of the IIIrd or Vth main groups or the Ist to VIIIth sub-groups of the periodic table;
R is an alkyl group with 1 to 6 carbon atoms;
L is a coordinating group with 2 to 6 complexing centres;
Sp is a spacer group or is absent;
Z is a polymerisable group;
a is a number from 2 to 20;
b is a number from 0 to 10;
c is a number from 1 to 30;
d, e independently of each other, are in each case a number from 0 to 30;
f is a number from 2 to 30,
and where any charge on the cluster (I) is satisfied by counterions.

2. Dental material according to Claim 1, **characterised in that** the variables have the following meanings:
M¹, M² = independently of each other, Ti and/or Zr;
R = an alkyl group with 1 to 4 carbon atoms, in particular 1 to 2 carbon atoms;
L = α-hydroxycarboxylate (-CH(OH) -COO⁻), α-aminocarboxylate (-CH(NH₂)-COO⁻), β-diketonate ([-C(-O⁻)=CH-C(=O)R^{K}]; with R^{K} = alkyl, preferably C₁ to C₆ alkyl, particularly preferably C₁ to C₃ alkyl, in particular methyl, sulphonate (-SO₃⁻) or phosphonate (-PO₃²⁻), particularly preferably carboxylate (-COO⁻);
Sp = an alkylene group with 1 to 18 carbon atoms, an oxyalkylene group with 1 to 18 carbon atoms and 0 to 6 oxygen atoms or an arylene group with 6 to 14 carbon atoms, the spacer Sp being able to comprise one or more, preferably 0 to 2 of the groups O, S, CO-O, O-CO, CO-NH, NH-CO, O-CO-NH, NH-CO-O and NH; particularly preferably, Sp is an alkylene group with 1 to 6, in particular 1 to 3 carbon atoms or is absent;
Z = an ethylenically unsaturated group, an epoxide, oxetane, vinyl ether, 1,3-dioxolane, spiroorthoester, particularly preferably a methacrylic and/or acrylic group;
a = 2 to 11;
b = 0 to 4.

3. Dental material according to Claim 2, **characterised in that** L-Sp-Z stands for acrylate, methacrylate, oleate, allyl acetoacetate and/or acetoacetoxyethyl methacrylate.

4. Dental material according to one of Claims 2 to 3, **characterised in that** the cluster comprises 1 to 4 kinds of ligands of the type L-Sp-Z.

5. Dental material according to one of Claims 2 to 4, **characterised in that** the cluster has a monodisperse weight distribution.

6. Dental material according to Claim 2 or 5, **characterised in that** the indices c to f assume values such that the positive charges of the metal or metals are completely satisfied.

7. Dental material according to one of Claims 2 to 6, **characterised in that** M¹ is the same as M².

8. Dental material according to one of Claims 2 to 7, **characterised in that** it comprises one or more further polymerisable components.

9. Dental material according to Claim 8, **characterised in that** the further polymcrisable component is a polymerisable polysiloxane, an ionically and/or radically polymerisable organic monomer or a mixture thereof.

10. Dental material according to one of Claims 2 to 9, **characterised in that** it comprises an initiator for ionic and/or radical polymerisation, filler and/or further additives.

11. Dental material according to one of the previous Claims, **characterised in that** it comprises, relative to its overall mass
(a) 5 to 90 wt.% of at least one cluster according to formula (I),
(b) 10 to 90 wt.% of a further polymerisable component,
(c) 0.1 to 5.0 wt.% polymerisation initiator,
and
(d) 0 to 90 wt.% filler.

12. Use of a cluster according to the general formula (I) as dental material or for the preparation of a dental material
[(M¹)ₐ(M²)_{b}O_{c}(OH)_{d}(OR)ₑ(L-Sp-Z)_{f}] (I)
in which
M¹, M² independently of each other, stand for a metal atom of the IIIrd or Vth main groups or the Ist to VIIIth sub-groups of the periodic table;
R is an alkyl group with 1 to 6 carbon atoms;
L is a coordinating group with 2 to 6 complexing centres;
Sp is a spacer group or is absent;
Z is a polymerisable group;
a is a number from 2 to 20;
b is a number from 0 to 10;
c is a number from 1 to 30;
d, e independently of each other, are in each case a number from 0 to 30;
f is a number from 2 to 30,
and where any charge on the cluster (I) is satisfied by counterions.

13. Use according to Claim 12 as adhesive, coating material, cement or filling material.

## Revendications

1. Matériau dentaire contenant un cluster selon la formule générale
[(M¹)ₐ(M²)_{b}O_{c}(OH)_{d}(OR)ₑ(L-Sp-Z)_{f}] (I)
dans laquelle
M¹, M² sont indépendamment l'un de l'autre un atome métallique du groupe principal III ou V ou des groupes secondaires I à VIII du système périodique de classification des éléments ;
R est un groupe alkyle comportant 1 à 6 atomes de carbone ;
L est un groupe de coordination avec 2 à 6 centres complexants ;
Sp est un groupe espaceur ou est absent ;
Z est un groupe capable de polymérisation ;
a est un nombre de 2 à 20 ;
b est un nombre de 0 à 10 ;
c est un nombre de 1 à 30 ;
d, e, sont indépendamment un nombre compris entre 0 et 30 ;
f est un nombre de 2 à 30,
dans lequel une charge éventuellement présente du cluster (I) est compensée par des contre-ions.

2. Matériau dentaire selon la revendication 1, **caractérisé en ce que** les variables ont les significations suivantes :
M¹, M² = indépendamment l'un de l'autre Ti et/ou Zr;
R = un groupe alkyle comportant 1 à 4 atomes de carbone, en particulier 1 à 2 atomes de carbone;
L = α-hydroxycarboxylate (-CH(OH)-COO⁻), α-aminocarboxylate (-CH(NH₂)-COO⁻), β-dicétonate ([C-(O⁻)=CH-C(=O)R^{k}) avec R^{k} = alkyle, ce préférence alkyle en C₁-C₆, de manière particulièrement préférée alkyle en C₁-C₃, en particulier méthyle, sulfonate (-SO₃⁻) ou phosphonate (-PO₃²⁻), de manière particulièrement préférée carboxylate (-COO⁻);
Sp = un groupe alkylène comportant 1 à 18 atomes de carbone, un groupe oxyalkylène comportant 1 à 18 atomes de carbone et 0 à 6 atomes d'oxygène ou un groupe arylène comportant 6 à 14 atomes de carbone, l'espaceur Sp pouvant contenir un ou plusieurs, de préférence 0 à 2 des groupes O, S, CO-O, O-CO, CO-NH, NH-CO, O-CO-NH, NH-CO-O, et NH ;
de manière particulièrement préférée un groupe alkylène comportant 1 à 6, en particulier 1 à 3 atomes de carbone ou est absent ;
Z = un groupe éthyléniquement insaturé, un groupe époxide, oxétane, vinyléther, 1,3-dioxolane, spiro-orthoester, de manière particulièrement préférée méthacryle et/ou acryle ;
a = 2 à 11
b = 0 à 4.

3. Matériau dentaire selon la revendication 2, **caractérisé en ce que** L-Sp-Z correspond à acrylate, méthacrylate, oléate, allylacéto-acétate et/ou acéto-acétoxyéthylméthacrylate.

4. Matériau dentaire selon l'une des revendications 2 à 3, **caractérisé en ce que** le cluster contient 1 à 4 types de ligands du type L-Sp-Z.

5. Matériau dentaire selon l'une des revendications 2 à 4, **caractérisé en ce que** le cluster présente une répartition de masse monodispersée.

6. Matériau dentaire selon la revendication 2 ou 5, **caractérisé en ce que** les indices c à f représentent des valeurs telles que les charges positives du ou des métaux sont complètement compensées.

7. Matériau dentaire selon l'une des revendications 2 à 6, **caractérisé en ce que** M¹ est égal à M².

8. Matériau dentaire selon l'une des revendications 2 à 7, **caractérisé en ce qu'**il contient un ou plusieurs composants polymérisables supplémentaires.

9. Matériau selon la revendication 8, **caractérisé en ce que** le composant polymérisable supplémentaire est un polysiloxane polymérisable, un monomère organique polymérisable par voie ionique et/ou radicalaire ou un mélange des deux.

10. Matériau dentaire selon l'une des revendications 2 à 9, **caractérisé en ce qu'**il contient un initiateur pour la polymérisation ionique et/ou radicalaire, une charge et/ou d'autres additifs.

11. Matériau dentaire selon l'une des revendications précédentes, **caractérisé en ce qu'**il contient, par rapport à sa masse totale,
(a) 5 à 90 % en poids au moins d'un cluster selon la formule (I)
(b) 10 à 90 % en poids de composants polymérisables supplémentaires,
(c) 0,1 à 5,0 % en poids d'initiateur de polymérisation,
et
(d) 0 à 90 % en poids de charge.

12. Utilisation d'un cluster de formule générale
[(M¹)ₐ(M²)_{b}O_{c}(OH)_{d}(OR)ₑ(L-Sp-Z)_{f}] (I)
dans laquelle
M¹, M² sont indépendamment l'un de l'autre un atome métallique du groupe principal III ou V ou des groupes secondaires I à VIII du système périodique de classification des éléments ;
R est un groupe alkyle comportant 1 à 6 atomes de carbone ;
L est un groupe de coordination avec 2 à 6 centres complexants ;
Sp est un groupe espaceur ou est absent ;
Z est un groupe capable de polymérisation ;
a est un nombre de 2 à 20 ;
b est un nombre de 0 à 10 ;
c est un nombre de 1 à 30 ;
d, e, sont indépendamment un nombre de 0 à 30 ;
f est un nombre de 2 à 30,
dans lequel une charge éventuellement présente du cluster (I) est compensée par des contre-ions, comme matériau dentaire ou pour la fabrication d'un matériau dentaire.

13. Utilisation selon la revendication 12 comme adhésif, matériau de revêtement, ciment ou matériau de remplissage.
